(19) Europäisches Patentamt
European Patent Office
Office européen des brevets

(11) Veröffentlichungsnummer: **0 483 631 A1**

(12) **EUROPÄISCHE PATENTANMELDUNG**

(21) Anmeldenummer: **91117896.0**

(22) Anmeldetag: **21.10.91**

(51) Int. Cl.$^5$: **C07D 261/04**, C07D 413/04, A61K 31/42

(30) Priorität: **01.11.90 DE 4034713**

(43) Veröffentlichungstag der Anmeldung: **06.05.92 Patentblatt 92/19**

(84) Benannte Vertragsstaaten: **BE CH DE DK ES FR GB GR IT LI NL SE**

(71) Anmelder: **BAYER AG**
**W-5090 Leverkusen 1 Bayerwerk(DE)**

(72) Erfinder: **Dyker, Gerald, Dr.**
**Sielkamp 2e**
**W-3300 Braunschweig(DE)**
Erfinder: **Lindner, Werner, Dr.**
**Märchenstrasse 39**
**W-5000 Köln 80(DE)**
Erfinder: **Harder, Achim, Dr. Dr.**
**Piccolministrasse 398**
**W-5000 Köln 80(DE)**
Erfinder: **Mencke, Norbert, Dr.**
**Grunder-Mühle 2**
**W-5090 Leverkusen 3(DE)**

(54) **3-Oxy-substituierte Isoxazoline, Verfahren zu ihrer Herstellung und ihre Verwendung zur Bekämpfung von Endparasiten.**

(57) Die vorliegende Erfindung betrifft neue 3-Oxysubstituierte Isoxazoline der Formel

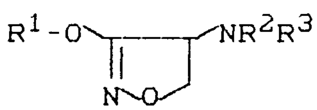

(I)

in welcher

$R^1$ für Wasserstoff oder gegebenenfalls substituiertes Alkyl steht,
$R^2$ für Wasserstoff, Acyl, Alkyl, Aryl steht,
$R^3$ für Wasserstoff, Acyl, Alkyl, Aryl steht
$R^2$ und $R^3$ gemeinsam mit den angrenzenden N-Atom für einen gesättigten Heterocyclen stehen,
wobei $R^1$, $R^2$ und $R^3$ nicht gleichzeitig für Wasserstoff stehen dürfen,
sowie die entsprechenden Tautomeren und Stereoisomeren dieser Verbindungen,
Verfahren zu ihrer Herstellung und ihre Verwendung als Endoparasitizide.

EP 0 483 631 A1

Die vorliegende Erfindung betrifft neue 3-Oxy-substituierte Isoxazoline, Verfahren zu ihrer Herstellung und ihre Verwendung zur Bekämpfung von Endoparasiten.

3-Chlorsubstituierte Isoxazoline und ihre Verwendung gegen Endoparasiten sind bereits bekannt geworden (US-PS 4 694 016, US-P 4 593 024). 3-Tolylthio-isoxazoline und ihre Verwendung gegen Endoparasiten sind bereits bekannt geworden (US-P 4 636 517).

Die Wirkung der bekannten Verbindungen befriedigt jedoch nicht in jedem Fall.

Die vorliegende Erfindung betrifft

1. Neue 3-Oxy-substituierte Isoxazoline der Formel I

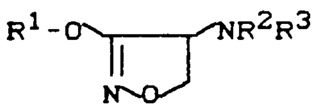

(I)

in welcher

$R^1$ für Wasserstoff oder gegebenenfalls substituiertes Alkyl steht,

$R^2$ für Wasserstoff, Acyl, Alkyl, Aryl steht,

$R^3$ für Wasserstoff, Acyl, Alkyl, Aryl steht

$R^2$ und $R^3$ gemeinsam mit dem angrenzenden N-Atom für einen gesättigten Heterocyclus stehen,

wobei $R^1$, $R^2$ und $R^3$ nicht gleichzeitig für Wasserstoff stehen dürfen,

sowie die entsprechenden Tautomeren und Stereoisomeren dieser Verbindungen.

2. Verfahren zur Herstellung der 3-Oxy-substituierten Isoxazoline der Formel I

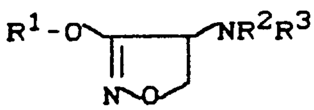

(I)

in welcher

$R^1$ für Wasserstoff oder gegebenenfalls substituiertes Alkyl steht,

$R^2$ für Wasserstoff, Acyl, Alkyl, Aryl steht,

$R^3$ für Wasserstoff, Acyl, Alkyl, Aryl steht

$R^2$ und $R^3$ gemeinsam mit dem angrenzenden N-Atom für einen gesättigten Heterocyclus stehen,

wobei $R^1$, $R^2$ und $R^3$ nicht gleichzeitig für Wasserstoff stehen dürfen,

dadurch gekennzeichnet, daß man

a) 3-Oxy-4-amino-substituierte Isoxazoline der Formel II

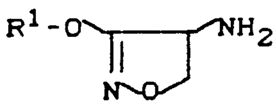

(II)

in welcher

$R^1$ die oben angegebene Bedeutung hat

mit mono- oder bifunktionellen Acylierungs-, Alkylierungs- oder Arylierungsmitteln umsetzt,

b) für den Fall, daB in Formel I $R^2$ für den Phthaloylrest und $R^3$ für Wasserstoff steht.

Verbindungen der Formel III

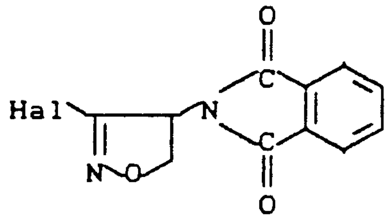

(III)

in welcher
Hal für Halogen steht,
mit Alkoholaten der Formel IV

$(R\text{-}O\text{-})_nM$ (IV)

in welcher
R für gegebenenfalls substituiertes Alkyl steht,
M für ein ein- oder mehrwertiges Metallkation steht,
n für ganze Zahlen von 1-3 steht,
umsetzt,
c) für den Fall, daß in Formel I $R^2$ und $R^3$ für Wasserstoff stehen, Verbindungen der Formel V

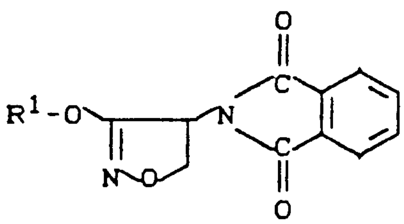

(V)

in welcher

$R^1$ die oben angegebene Bedeutung hat,
mit Alkylhydrazinen der Formel VI

$H_2N\text{-}NH\text{-}R^4$

in welcher
$R^4$ für $C_{1-4}$-Alkyl steht
umsetzt.
3. Neue Verbindungen der Formel V

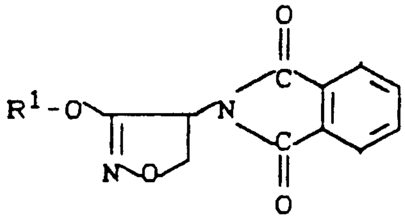

(V)

in welcher
$R^1$ die unter 1 angegebene Bedeutung hat.
4. Verfahren zur Herstellung der Verbindungen der Formel V

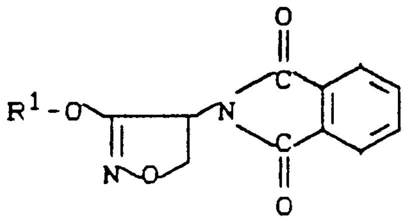

(V)

in welcher

$R^1$ die unter 1 angegebene Bedeutung hat,

dadurch gekennzeichnet, daß man Verbindungen der Formel III

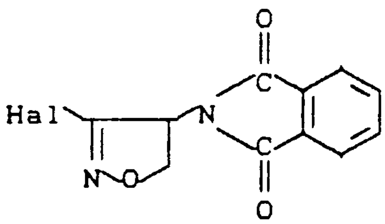

(III)

in welcher

Hal für Halogen steht,

mit Alkoholaten der Formel IV

$(RO)_nM$

in welcher

R, M, n die unter 1 angegebene Bedeutung haben,

umsetzt,

Bevorzugt sind Verbindungen der Formel I, in welcher das C-Atom 4 des Isoxazolinringes in der R-Konfiguration vorliegt.

Bevorzugt sind Verbindungen der Formel I, in welcher

$R^1$ für Wasserstoff oder $C_{1-12}$-Alkyl steht, das gegebenenfalls substituiert ist durch OH, CN, Halogen, insbesondere Fluor oder Chlor, $C_1$-$C_4$-Alkoxy, $C_{1-4}$-Alkylthio, $C_{3-6}$-Cycloalkyl, 5-6-gliedrige gesättigte oder ungesättigte heterocyclische Ringe die N,O oder S als Heteroatome enthalten können wie z.B. Furan, Tetrahydrofuran, Tetrahydrofuran, Thiophen, Pyrrol, Pyridin, Pyrimidin; ferner durch Phenyl das seinerseits substituiert sein kann durch $C_{1-4}$-Alkyl, $C_{1-4}$-Alkoxy, $C_{1-4}$-Alkylthio, $C_{1-4}$-Halogenalkyl, $C_{1-4}$-Halogenalkoxy, $C_{1-4}$-Halogenalkylthio, $C_{1-4}$-Alkylendioxy, $C_{1-4}$-Halogenalkylendioxy, OH, CN, Halogen, Carboxyl, $C_{1-4}$-Alkoxycarbonyl, $C_{1-4}$-Alkoxy-$C_{1-4}$-alkyl, Hydroxy-$C_{1-4}$-alkyl, Hydroxy-$C_{1-4}$-alkoxy, Phenoxy, Phenylthio,

$R^2$ für Wasserstoff, gegebenenfalls substituiertes $C_{1-8}$-Alkyl, gegebenenfalls substituiertes Phenyl, Acylreste wie $C_{1-8}$-Alkylcarbonyl das gegebenenfalls subsituiert ist, $C_{1-8}$-Alkylsulfonyl das gegebenenfalls substituiert ist, $C_{1-8}$-Alkylsulfonyl das gegebenenfalls substituiert ist, Arylcarbonyl, insbesondere Benzoyl, das gegebenenfalls substituiert ist, Arylsulfonyl insbesondere Phenylsulfonyl, das gegebenenfalls substituiert ist, Aminocarbonyl, Mono- oder Di-$C_{1-4}$-alkylaminocarbonyl, $C_{1-4}$-Alkyl-arylaminocarbonyl, Mono- oder -Diarylaminocarbonyl (wobei als Arylrest insbesondere Phenyl genannt sei und die Alkyl- oder Arylreste gegebenenfalls substituiert sein können), $C_{1-4}$-Alkoxycarbonyl, das gegebenenfalls substituiert ist, Aryloxycarbonyl, insbesondere Phenoxycarbonyl, das gegebenenfalls substituiert ist, $C_{1-4}$-Alkyl- oder Aryl-Amino-carbonyliminoalkyl oder -aryl, das gegebenenfalls in den Alkyl- oder Arylteilen substituiert ist und wobei als Aryl insbesondere Phenyl steht. Als Substituenten kommen die bei $R^1$ genannten Substituenten des Alkylrestes in Frage,

$R^3$ für die bei $R^2$ genannten Reste steht,

$R^2$ und $R^3$ gemeinsam mit dem angrenzenden N-Atom für einen 5-6-gliedrigen, gesättigten oder ungesättigten Heterocyclus stehen wie z.B. Pyrrol, Pyrrolidin, Piperidin, Morpholin.

Besonders bevorzugt sind Verbindungen der Formel I, in welcher

$R^1$ für Wasserstoff, $C_{1-8}$-Alkyl steht, das gegebenenfalls substituiert ist durch Halogen, insbesondere Fluor oder Chlor, $C_{1-4}$-Alkoxy, insbesondere Methoxy, Cyclopropyl, Tetrahydrofuran, Furan, Tetrahydrofuranon, Pyrrol, Thiophen, Pyridin, Phenyl, ferner für Benzyl steht, das gegebenenfalls substituiert ist durch Halogen wie Fluor oder Chlor, $C_{1-4}$-Alkoxy wie Methoxy, Ethoxy, Methylendioxy, Ethylendioxy, $C_{1-4}$-Alkyl wie Methyl, Ethyl, $C_{1-4}$-Halogenalkyl wie Trifluormethyl, Carbonyl-$C_{1-4}$-alkoxy wie Methoxycarbonyl, $C_{1-4}$-Halogenalkylthio insbesondere Trifluormethylthio, $C_{1-4}$-Halogenalkoxy insbesondere Trifluormethoxy.

$R^2$ für Wasserstoff oder gegebenenfalls substituiertes Benzoyl steht, wobei als Substituenten die bei $R^1$ genannten in Frage kommen und insbesondere Carboxyl (COOH) genannt sei.

Im einzelnen seien folgende Verbindungen der Formel I genannt:

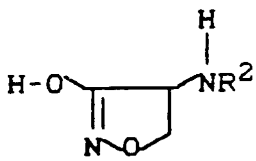

| $R^2$ |
| --- |
| 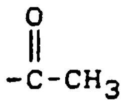 |
| 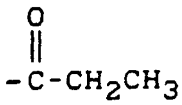 |
| 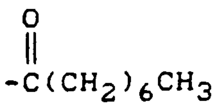 |
| 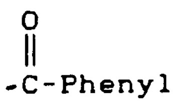 |
| 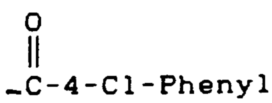 |
| 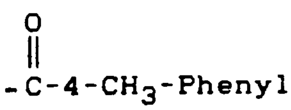 |
| 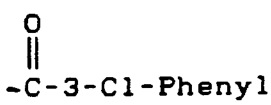 |
| 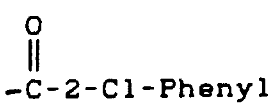 |

| $R^2$ |
|---|
| $-CO-3-CH_3$-Phenyl |
| $-CO-2-CH_3$-Phenyl |
| $-CO-4-SCF_3$-Phenyl |
| $-CO-4-CF_3$-Phenyl |
| $-CO-4-OCF_3$-Phenyl |
| $-CO-3,4-Cl_2$-Phenyl |
| $-CO-2,4-Cl_2$-Phenyl |
| $-CO-4-OCH_3$-Phenyl |
| $-CO-3-OCH_3$-Phenyl |
| $-CO-2-OCH_3$-Phenyl |
| $-CO-4-SCH_3$-Phenyl |
| $-CO-4-SO_2CH_3$-Phenyl |
| $-SO_2-CH_3$ |
| $-SO_2-CF_3$ |
| $-SO_2$-Phenyl |
| $-SO_2-4-CH_3$-Phenyl |
| $-SO_2$-4-Cl-Phenyl |
| $-CO-OCH_3$ |
| $-CO-OC_2H_5$ |
| -CO-O-Phenyl |
| -CO-O-4-Cl-Phenyl |
| $-CO-O-CH_2$-Phenyl |
| $-CO-NHCH_3$ |
| $-CO-NHC_2H_5$ |
| $-CO-NH(CH_2)_6CH_3$ |
| -CO-NH-Phenyl |
| -CO-NH-4-Cl-Phenyl |
| -CO-NH-3-Cl-Phenyl |
| -CO-NH-2-Cl-Phenyl |
| $-CO-NH-4-CH_3$-Phenyl |

| $R^2$ |
|---|
| $-CO-NH-3-CH_3$-Phenyl |
| $-CO-NH-2-CH_3$-Phenyl |
| $-CO-NH-3,4-Cl_2$-Phenyl |
| $-CO-NH-2,4-Cl_2$-Phenyl |
| $-CO-NH-4-SCF_3$-Phenyl |
| $-CO-NH-4-OCF_3$-Phenyl |
| $-CO-NH-2,3-(CH_3)_2$-Phenyl |
| $-CO-NH-3-Cl, 4-CF_3$-Phenyl |
| $-CO-NH-4-SCH_3$-Phenyl |
| $-CO-NH-4-SO_2CH_3$-Phenyl |
| $-CO-NH-4-NO_2$-Phenyl |
| -CO-NH-(4-Phenoxy)-Phenyl |
| $-CNCH_3-NHCH_3$ |
| $-CH_3$ |
| $-C_2H_5$ |
| $-C_3H_7$ |
| $-i-C_3H_7$ |
| $-C_6H_{13}$ |
| $-CH_2$-Phenyl |
| $-CH_2$-4-Cl-Phenyl |

Setzt man zur Herstellung der Verbindungen der Formel I nach Verfahren 2a 3-Methoxy-4-amino-4,5-dihydroisoxazol, so läßt sich das Verfahren in Abhängigkeit von den verwendeten Acylierungs-, Alkylierungs- oder Arylierungsmitteln durch folgende Formelschemata wiedergeben:

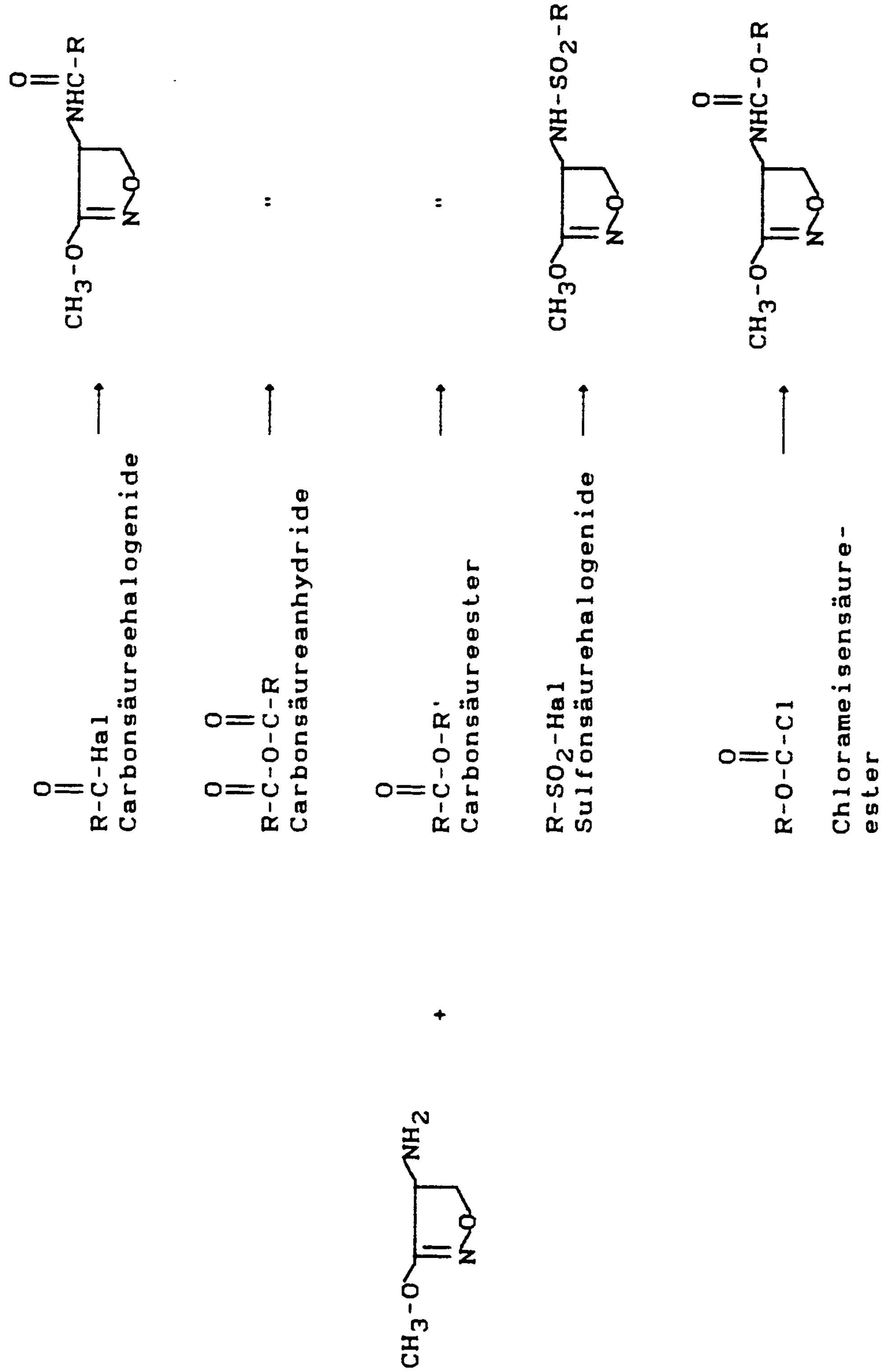
Carbonsäureehalogenide
Carbonsäureanhydride
Carbonsäureester
Sulfonsäurehalogenide
Chlorameisensäure-
ester

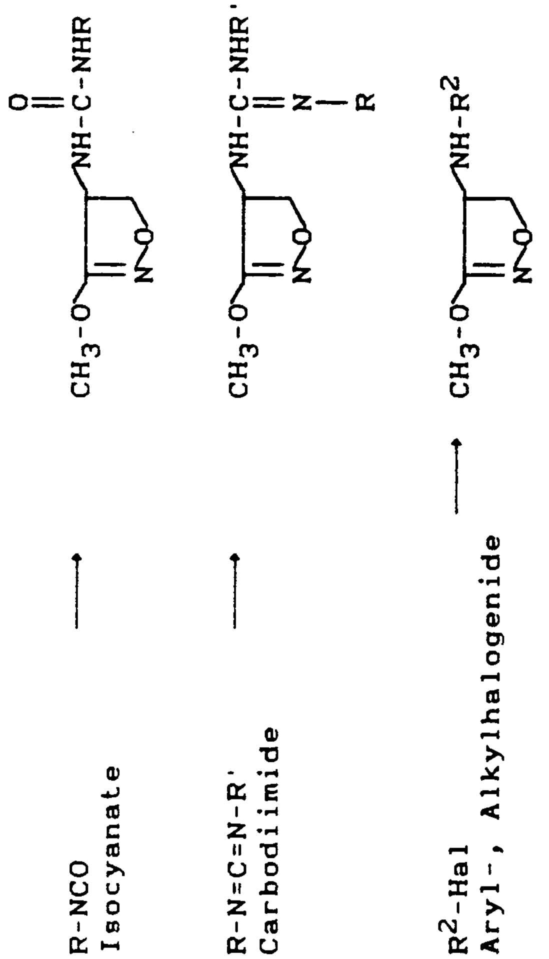


Bevorzugt werden Verbindungen der Formel I eingesetzt, in welcher $R^1$ die bei den Verbindungen der Formel I als bevorzugt genannten Reste besitzt.

Als Acylierungsmittel seien bevorzugt genannt: Carbonsäurehalogenide, insbesondere Chloride, Carbonsäureanhydride und Carbonsäureester, insbesondere $C_{1-4}$-Alkylester wie Methyl- oder Ethylester. Es seien bevorzugt Verbindungen genannt, durch die die bei $R^2$ genannten bevorzugten Acylreste eingführt werden.

Im einzelnen seien genannt:

$CH_3COCl$
$C_2H_5COCl$
4-Cl-Phenyl-COCl
$(CH_3CO)_2O$
4-$CH_3$-Phenyl-CO-O-$C_2H_5$
4-Cl-Phenyl-$CH_2$-COOEt
$(CF_3CO)_2O$
Cyclopropylcarbonsäurechlorid
Cyclohexancarbonsäurechlorid

Sulfonsäurehalogenide insbesondere Chloride wie $C_{1-4}$-Alkylsulfonsäurechloride oder gegebenenfalls substituiertes Phenylsulfonsäurechlorid.

$CH_3SO_2Cl$
Phenyl-$SO_2Cl$
4-Cl-Phenyl-$SO_2Cl$
4-$CH_3$-Phenyl-$SO_2Cl$

Chlorameisensäureester insbesondere

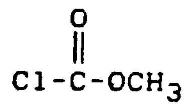

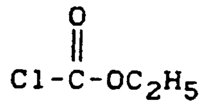

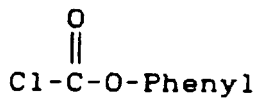

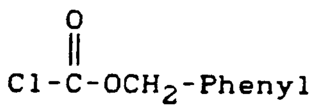

Alkylisocyanate oder Phenylisocyanate

Gegebenenfalls substituierte $C_{1-4}$-Alkylisocyanate oder Phenylisocyanate, insbesondere $CH_3NCO$, $C_2H_5NCO$, i-$C_3H_7NCO$, $CH_3$-Hexyl-NCO, Phenyl-NCO, 4-Cl-Phenyl-NCO, 4-$CH_3$-Phenyl-NCO, 3-Cl-Phenyl-NCO, 3-$CH_3$-Phenyl-NCO, 2,4-$Cl_2$-Phenyl-NCO, 4-$OCF_3$-Phenyl-NCO, 3,4-$Cl_2$-Phenyl-NCO, Phenyl-$SO_2$-NCO.

Carbodiimide wie insbesondere Dicyclohexylcarbodiimid , Di-Tolylcarbodiimid, Diisopropylcarbodiimid.

Die Acylierungsreaktionen werden in an sich bekannter Weise durchgeführt. Die Verbindungen der Formel II und die Acylierungsmittel werden in äquimolarem Verhältnis eingesetzt. Es kann auch mit einem Überschuß der einen oder anderen Komponente gearbeitet werden. Die Reaktionen erfolgen beim Temperaturen von 0-200 ° C, bevorzugt bei 50-150 ° C. Die Reaktionen können in Gegenwart von Verdünnungsmitteln durchgeführt werden.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Die Reaktion kann in Gegenwart einer Base durchgeführt werden.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecen(DBU), 1,4-Diazabicyclo(2,2,2)octan (DABCO), Diazabicyclo(3,2,0)nonen (DBN), Ethyl-diisopropylamin.

Als Alkylierungsmittel seien bevorzugt genannt gegebenenfalls subsituierte $C_{1-8}$-Alkylhalogenide, insbesondere Chloride, Bromide, Jodide.

Im einzelnen seien genannt: $CH_3I$, $C_2H_5Br$, i-$C_3H_7I$, $C_3H_7I$, $C_6H_{13}I$, Phenyl-$CH_2I$, 4-Cl-Phenyl-$CH_2$-Cl.

Als Arylierungsmittel seien bevorzugt genannt: 4-Cl-Nitrobenzol, 3,4,5-Trichlornitrobenzol.

Das Verfahren wird durchgeführt, indem man eine Verbindung der Formel II in Gegenwart einer Base und eines Verdünnungsmittels mit dem Alkylierungs- bzw. Arylierungsmittel umsetzt. Als Verdünnungsmittel können alle inerten organischen Lösungsmittel eingesetzt werden, die bereits weiter oben genannt sind.

Das Verfahren wird in Gegenwart von Basen durchgeführt. Als bevorzugte Basen seien genannt die Alkalihydroxide wie Natriumhydroxid, Alkalialkoholate wie Natriummethylat oder Kaliumbutanolat, Metallhydride wie Natriumhydrid oder organische Basen wie 1,8-Diazabicyclo[5,40]undec-7-en (DBU).

Das Verfahren wird durchgeführt bei Normaldruck und Temperaturen zwischen 20 ° C und 140 ° C.

Die Reaktion wird durchgeführt indem man äquimolare Mengen der Verbindung der Formel II und Base zusammengibt, dieses Gemisch mit einer äquimolaren Menge der Alkylierungs- oder Arylierungsmittel versetzt und auf die Reaktionstemperatur erhitzt.

Setzt gemäß Verfahren 2b man zur Herstellung der Verbindungen der Formel I in der der Rest $R^2$ für den Phthaloylrest und $R^3$ für Wasserstoff steht als Verbindung der Formel III 3-Chor-4,5-dihydrophthalimidoisoxazolin und als Alkoholat der Formel IV Kaliumethylat ein, läßt sich die Reaktion durch folgendes Formelschema wiedergeben:

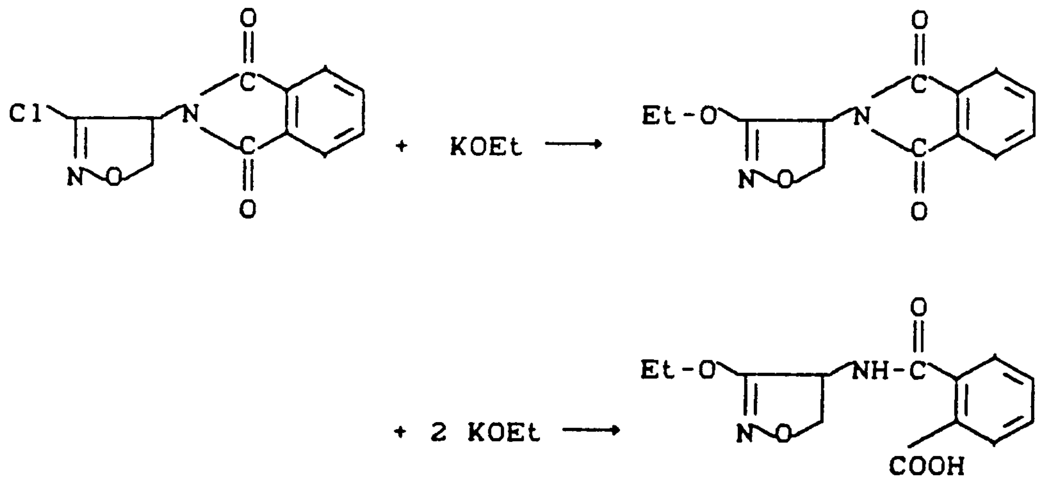


Bevorzugt wird als Verbindung der Formel III die 3-Chlor-Verbindung eingesetzt.

Als Alkoholate der Formel IV werden bevorzugt eingesetzt Alkali- und Erdalkalialkoholate in denen R für die bei $R^1$ als bevorzugt angegeben ist.

Im einzelnen seien genannt: Na-$OC_2H_5$, Na-$OC_6H_{13}$, K-O-i$C_3H_7$, Na-$OCH_2$-Phenyl, Na-O-Phenyl, 4-Cl-Phenyl-O-Na.

Wird die Verbindung der Formel III mit dem Alkoholat in etwa äquimolaren Verhältnis umgesetzt, entsteht das entsprechende 3-Oxy-4-phthalimido-isoxazolin.

Die Reaktion wird bei Temperaturen von 0-100 °C bevorzugt 15-25 °C durchgeführt. Als Verdünnungsmittel dienen die dem Alkoholaten zugrundeliegenden Alkohole oder unter den Reaktionsbedingungen inerte organische Verdünnungsmittel wie z.B. die weiter oben bei Verfahren 2a genannten.

Nach beendeter Reaktion wird das Lösungsmittel abdestilliert, der Rückstand mit Wasser versetzt und mit einem nicht mit Wasser mischbaren Lösungsmittel extrahiert. Nach Entfernen des Extraktionsmittel erhält man die gewünschte Verbindung der Formel I`

Wird die Verbindung der Formel II mit Überschuß an Alkoholat umgesetzt, entsteht das entsprechende 3-Oxy-4-phthalsäuremonoamid-4,5-dihydroisoxazolin. Überraschenderweise entsteht bei dieser Reaktion nicht der entsprechende Phthalsäureamidester.

Verbindung der Formel III und Alkoholat werden im Verhältnis 1:2 bis 1:10 bevorzugt 1:2 bis 1:4 eingesetzt.

Die Reaktion wird wie oben angegeben durchgeführt.

Nach beendeter Reaktion wird das Lösungsmittel abdestilliert, der Rückstand mit Wasser versetzt und angesäuert. Dabei fällt die gewünschte Verbindung der Formel I aus und kann abfiltriert werden.

Setzt man gemäß Verfahren 2c zur Herstellung der Verbindungen der Formel I, in welcher $R^2$ und $R^3$ für Wasserstoff stehen, als Verbindung der Formel V 3-Phenoxy-4-phthalimido-4,5-dihydroisoxazol ein, so läßt sich die Reaktion durch folgendes Formelschema wiedergeben:

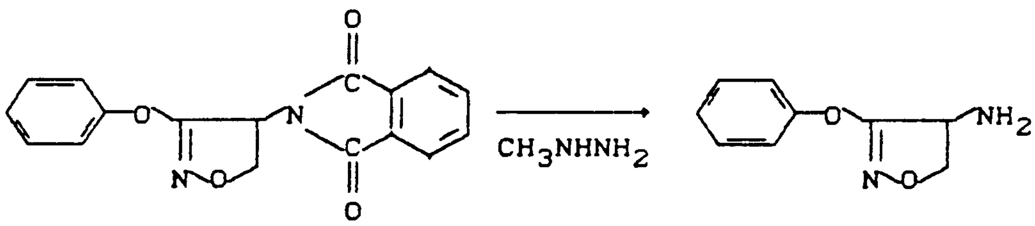


Als Verbindungen der Formel V werden bevorzugt diejenigen eingesetzt, in denen der Rest $R^1$ die bei den Verbidungen der Formel I angegebene bevorzugte Bedeutung besitzt. Im einzelnen seien genannt:

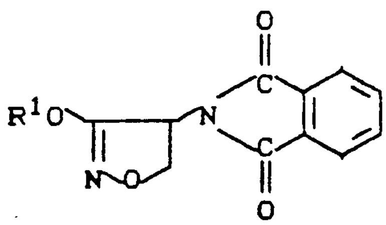

| $R^1$ |
|---|
| $iC_3H_7$ |
| $C_2H_5$ |
| $4-OCH_3$-Phenyl |
| 4-Cl-Phenyl |
| sek.-$C_4H_9$ |
| $(CH_2)_7-CH_3$ |
| $CH_2$-4-Cl-Phenyl |
| $CH_2$-Cy-Propyl |
| $-CH_2-4-CO_2CH_3$-Phenyl |
| $-CH_2-3,4-Cl_2$-Phenyl |
| $-CH_2-2,4-Cl_2$-Phenyl |
| $-CH_2-CF_3$ |
| $-CH_2-4-OCH_3$-Phenyl |
| 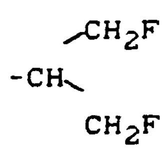 |
| $-CH_2-4-CF_3$-Phenyl |
| $-CH_2CCl_3$ |

Als Alkylhydrazine der Formel VI werden bevorzugt eingesetzt: Methylhydrazin.

Die Verbindungen der Formeln V und VI werden im Verhältnis 1:1 bis 1:20 bevorzugt 1:5 bis 1:10 eingesetzt.

Die Reaktion wird bei Temperaturen von 0-100 ° C bevorzugt 15-25 ° C durchgeführt.

Als Verdünnungsmittel kommen die bei Verfahren 2a angegebenen Verdünnungsmitteln in Frage. Besonders erwähnt sei als Verdünnungsmittel ein Gemisch aus Methanol und Tetrahydrofuran.

Die Verbindungen der Formel V stellen eine bevorzugte Gruppe unter den Verbindungen der Formel I dar. Ihre Herstellung erfolgt nach Verfahren 4, das identisch ist mit Verfahrenb 2b bei dem die Verbindung der Formel III mit in etwa äquimolaren Mengen Alkoholat umgesetzt wird. Die Verbindungen der Formel V dienen sowohl als Wirkstoffe als auch als Zwischenprodukte zur Herstellung weiterer Verbindungen der Formel I. Die Verbindungen der Formel III sind bekannt z.B. aus US-P 4 636 517.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp, Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird, Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$,Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate. Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm bis 20 Gewichtsprozent, bevorzugt von 0,1 bis 10 Gewichtsprozent.

Zubereitungen, die vor Anwendung verdünnt werden, enthalten den Wirkstoff in Konzentrationen von 0,5 bis 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

<u>Beispiel A</u>

In vivo Nematodentest

Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 2 | 25 |
| 3 | 25 |
| 1 | 25 |

<u>Beispiel B</u>

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Würmer vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 1 | 10 |
| 2 | 10 |
| 3 | 10 |

Herstellungsbeispiele

Beispiel 1

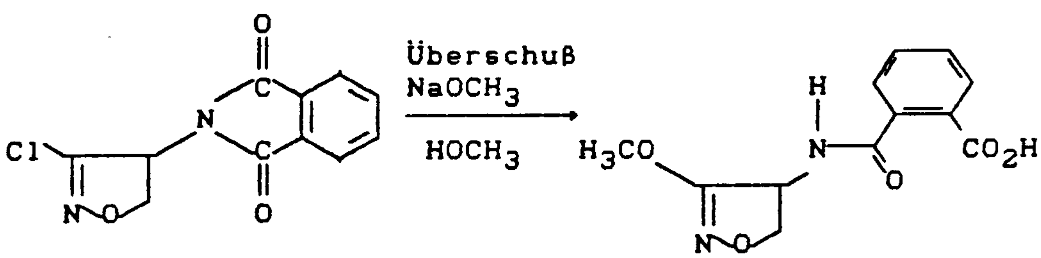


(R)-N-(4,5-Dihydro-3-methoxy-4-isoazolyl)-phthalamidsäure

10 g (40 mmol) (R)-3-Chlor-4,5-dihydro-4-phthalimidoisoxazol und 9,1 g (160 mmol) Natriummethanolat werden in 50 ml wasserfreiem Methanol 1 Stunde bei Raumtemperatur umgesetzt. Das Lösungsmittel destilliert man ab, löst den Rückstand in 100 ml Wasser und säuert mit verdünnter Salzsäure an. Nach Extrahieren mit dreimal 30 ml Essigsäureethylester und Trocknen der vereinigten organischen Extrakte mit Natriumsulfat engt man ein und trocknet den Rückstand bei 50 °C im Vakuum. Ausbeute: 6,1 g (58 %) der Phthalamidsäure als farbloser Feststoff mit Zersetzungspunkt 152 °C.

Beispiel 2

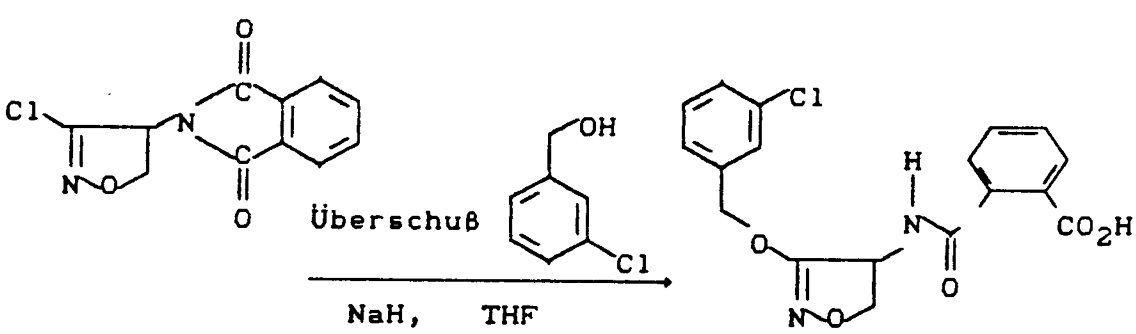


(R)-N-[4-5-Dihydro-3-(-3-chlorphenyl)-methoxy-4-isoxazolyl]-phthalamidsäure

5,0 g (20 mmol) (R)-3-Chlor-4,5-dihydro-4-phthalimidoisoxazol, 11 g (80 mmol) 3-Chlorbenzylalkohol und 1,9 g (80 mmol) Natriumhydrid werden in 30 ml wasserfreiem Tetrahydrofuran 16 Stunden bei Raumtemperatur umgesetzt. Das Lösungsmittel destilliert man ab und nimmt den Rückstand in 250 ml Wasser auf. Nach Extrahieren mit 50 ml Dichlormethan wird die wäßrige Phase mit verdünnter Salzsäure

angesäuert und der Niederschlag abfiltriert. Zur Reinigung löst man das Rohprodukt in 200 ml 20 %iger Natronlauge und fällt mit 250 ml 15 %iger Salzsäure. Der Niederschlag wird abfiltriert, mit wenig Wasser gewaschen und bei 50 ° C im Vakuum getrocknet. Ausbeute: 7,1 g (95 %) der Phthalamidsäure als farbloser Feststoff mit Zersetzungspunkt 149 ° C.

Analog werden hergestellt:

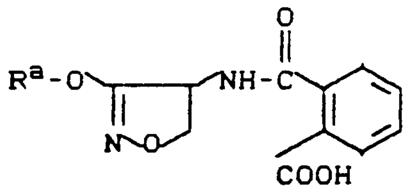

| Bsp.-Nr. | $R^a$ | Fp° C |
|---|---|---|
| 3 | 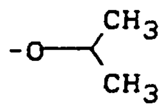 | 161° C |
| 4 | 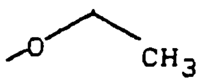 | 176° C |
| 5 | 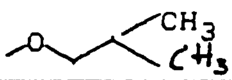 | 136° C |
| 6 | 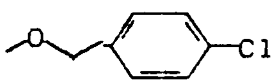 | 180° C |
| 7 | 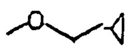 | 146° C |
| 8 | 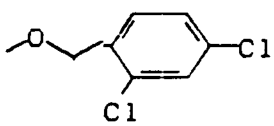 | 201° C |
| 9 | 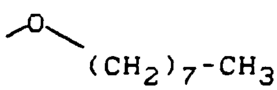 | 80° C |
| 10 | 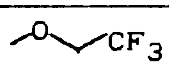 | |
| 11 | 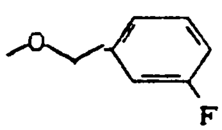 | 136° C |
| 12 | 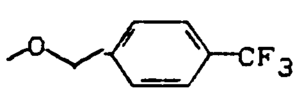 | 147° C |

(Fortsetzung)

| Bsp.-Nr. | $R^a$ | Fp° C |
|---|---|---|
| 13 | 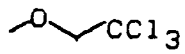 | 138° C |
| 14 | 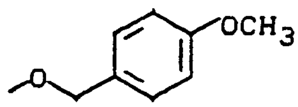 | 145° C |
| 15 | 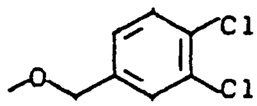 | 173° C |
| 16 | 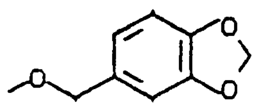 | 170° C |
| 17 | 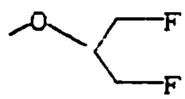 | 171° C |
| 18 | 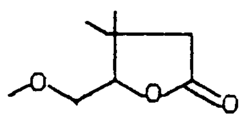 | Öl |
| 19 | 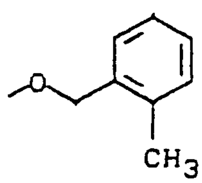 | 179° C |
| 20 | 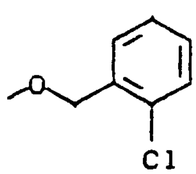 | 152° C |
| 21 | 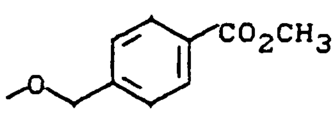 | 185° C |

(Fortsetzung)

| Bsp.-Nr. | $R^a$ | Fp° C |
|---|---|---|
| 22 | 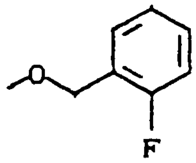 | 173° C |
| 23 | 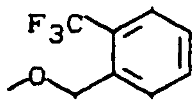 | 101° C |
| 24 | 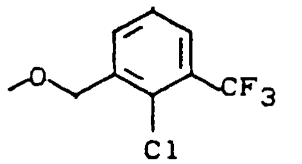 | 167° C |
| 25 | 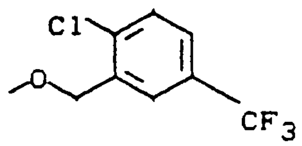 | 161° C |
| 26 | 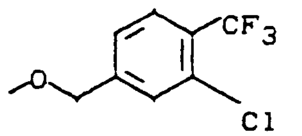 | 192° C |
| 27 | 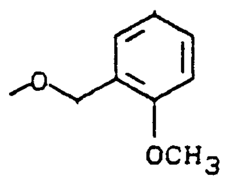 | 153° C |
| 28 | 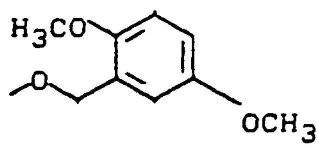 | 133° C |
| 29 | 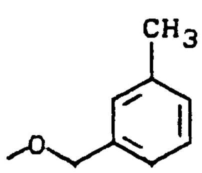 | 150° C |

(Fortsetzung)

| Bsp.-Nr. | $R^a$ | Fp° C |
|---|---|---|
| 30 | 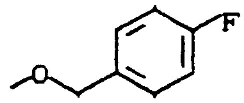 | 178° C |
| 31 | 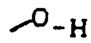 | |

Beispiel 32

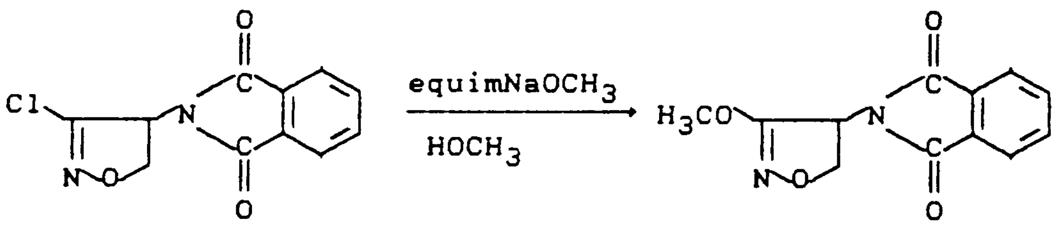


(R)-4,5-Dihydro-3-methoxy-4-phthalimido-isoxazol

Zu 40 g (0,16 mol) (R)-3-Chlor-4,5-dihydro-4-phthalimido-isoxazol in 150 ml trockenem Methanol werden 8,6 g (0,16 mol) Natriummethanolat in 50 ml Methanol innerhalb von 1 Stunde bei Raumtemperatur zugetropft.

Nach 15 h Nachrühren engt man ein, nimmt den Rückstand in 400 ml Wasser auf und extrahiert dreimal mit 100 ml Essigsäureethylester.

Die vereinigten organischen Extrakte werden mit Natriumsulfat getrocknet und eingeengt. Den Rückstand trocknet man bei 50 ° C im Vakuum und erhält 31,9 g (81 %) (R)-4,5-Dihydro-3-methoxy-4-phthalimido-isoxazol als farblosen Feststoff mit Schmp. 122 ° C.

Beispiel 33

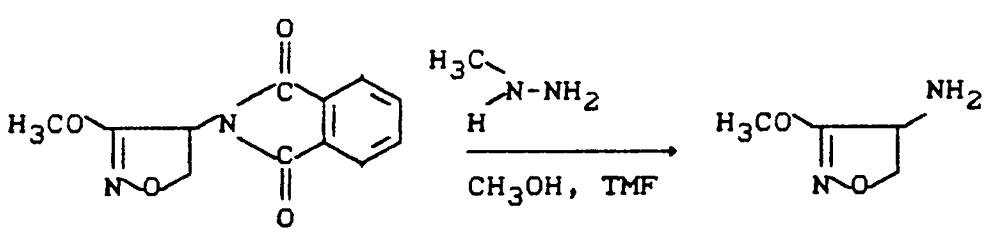


(R)-4-Amino-4,5-dihydro-3-methoxy-isoxazol

Eine Lösung von 26,0 g (105 mmol) (R)-4,5-Dihydro-3-methoxy-4-phthalimido-isoxazol und 48,8 g (1.06 mol) Methylhydrazin in 500 ml Methanol/Tetrahydrofuan (1:1) wird nach 5 h bei Raumtemperatur eingeengt und der Rückstand dreimal mit 100 ml Ether extrahiert. Die vereinigten Etherphasen engt man ein und reinigt den Rückstand säulenchromatographisch über Kieselgel mit Dichlormethan/Methanol (1:1) als Eluens.

Ausb. 7,4 g (60 %) (R)-4-Amino-4,5-dihydro-3-methoxyisoxazol als farblose Flüssigkeit mit $n_D^{20}$ = 1.4792.

**Patentansprüche**

**1.** 3-Oxy-substituierte Isoxazoline der Formel )

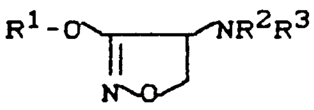

(I)

in welcher

| | |
|---|---|
| $R^1$ | für Wasserstoff oder gegebenenfalls substituiertes Alkyl steht, |
| $R^2$ | für Wasserstoff, Acyl, Alkyl, Aryl steht, |
| $R^3$ | für Wasserstoff, Acyl, Alkyl, Aryl steht |
| $R^2$ und $R^3$ | gemeinsam mit dem angrenzenden N-Atom für einen gesättigten Heterocyclus stehen, |

wobei $R^1$, $R^2$ und $R^3$ nicht gleichzeitig für Wasserstoff stehen dürfen,

sowie die entsprechenden Tautomeren und Stereoisomeren dieser Verbindungen.

**2.** Verfahren zur Herstellung der 3-Oxy-substituierten Isoxazoline der Formel I

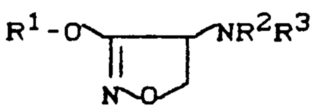

(I)

in welcher

| | |
|---|---|
| $R^1$ | für Wasserstoff oder gegebenenfalls substituiertes Alkyl steht, |
| $R^2$ | für Wasserstoff, Acyl, Alkyl, Aryl steht, |
| $R^3$ | für Wasserstoff, Acyl, Alkyl, Aryl steht |
| $R^2$ und $R^3$ | gemeinsam mit dem angrenzenden N-Atom für einen gesättigten Heterocyclus stehen, |

wobei $R^1$, $R^2$ und $R^3$ nicht gleichzeitig für Wasserstoff stehen dürfen,

dadurch gekennzeichnet, daß man

a) 3-Oxy-4-amino-substituierte Isoxazoline der Formel II

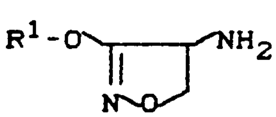

(II)

in welcher

$R^1$ die oben angegebene Bedeutung hat

mit mono- oder bifunktionellen Acylierungs-, Alkylierungs- oder Arylierungsmittel umsetzt,

b) für den Fall, daß in Formel I $R^2$ für den Phthaloylrest und $R^3$ für Wasserstoff steht.

Verbindungen der Formel III

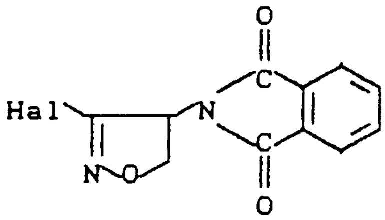

(III)

in welcher

Hal für Halogen steht,

mit Alkoholaten der Formel IV

$(R\text{-}O\text{-})_nM$ (IV)

in welcher
R für gegebenenfalls substituiertes Alkyl steht,
M für ein ein- oder mehrwertiges Metallkation steht,
n für ganze Zahlen von 1-3 steht,
umsetzt,
c) für den Fall, daß in Formel I $R^2$ und $R^3$ für Wasserstoff stehen, Verbindungen der Formel V

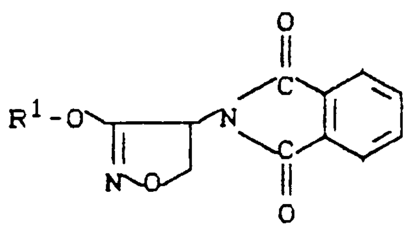

(V)

in welcher
$R^1$ die oben angegebene Bedeutung hat,
mit Alkylhydrazinen der Formel VI

$H_2N\text{-}NH\text{-}R^4$

in welcher
$R^4$ für $C_{1-4}$-Alkyl steht
umsetzt.

**3.** Neue Verbindungen der Formel V

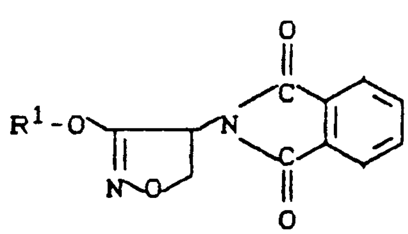

(V)

in welcher

$R^1$ die unter 1 angegebene Bedeutung hat.

**4.** Verfahren zur Herstellung der Verbindungen der Formel III

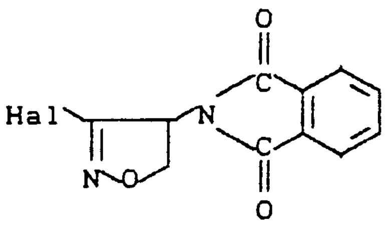

(III)

in welcher

Hal für Halogen steht,

mit Alkoholaten der Formel IV

$(RO)_nM$

in welcher
R, M, n die in Anspruch 2 angebene Bedeutung haben,
umsetzt.

**5.** Verwendung von 3-oxysubstituierten Isoxazolinen der Formel I gemäß Anspruch 1 zur Bekämpfung von Endoparasiten.

**6.** Endoparasitizide Mittel, gekennzeichnet durch einen Gehalt an mindestens einem 3-oxysubstituierten Isoxazolin der Formel (I) gemäß Anspruch 1.

**7.** Verfahren zur Herstellung von endoparasitiziden Mitteln, dadurch gekennzeichnet, daß man 3-oxysubstituierten Isoxazoline der Formel (I) gemäß Anspruch 1 mit Streckmitten und/oder oberflächenaktiven Mitteln vermischt.

**8.** Verwendung von 3-oxysubstituierten Isoxazolinen der Formel (I) gemäß Anspruch 1 zur Herstellung von endoparasitiziden Mitteln.

Europäisches Patentamt

# EUROPÄISCHER RECHERCHENBERICHT

Nummer der Anmeldung

EP 91 11 7896
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | US-A-2 815 348 (WALLACE F. RUNGE)<br>* das ganze Dokument *<br>--- | 1 | C07D261/04<br>C07D413/04<br>A61K31/42 |
| X | US-A-2 845 432 (FREDERICK A. KUEHL)<br>* das ganze Dokument *<br>--- | 1,5-8 | |
| X | US-A-2 967 866 (EDWARD B. HODGE)<br>* das ganze Dokument *<br>--- | 1,5-8 | |
| X | DE-A-2 436 959 (MERCK AND CO.)<br>* das ganze Dokument *<br>--- | 1,5-8 | |
| X | DE-A-2 436 960 (MERCK AND CO.)<br>* das ganze Dokument *<br>--- | 1,5-8 | |
| D | US-A-4 593 024 (JING-JONG LU) | | |
| X | * Spalte 4; Beispiel 1 * | 1 | |
| A | * Ansprüche *<br>--- | 5-8 | |
| X | JOURNAL OF MEDICINAL CHEMISTRY.<br>Bd. 23, Nr. 1, Januar 1980, WASHINGTON US<br>Seiten 6 - 8;<br>N.P. JENSEN ET AL: 'Use of Acetylacetone to Prepare a Prodrug of Cycloserine'<br>* das ganze Dokument *<br>--- | 1,5-8 | RECHERCHIERTE SACHGEBIETE (Int. Cl.5 )<br><br>C07D |
| X | JOURNAL OF MEDICINAL CHEMISTRY.<br>Bd. 13, Nr. 5, Mai 1970, WASHINGTON US<br>Seiten 1013 - 1015;<br>C. H. STAMMER ET AL: 'Cycloserine Derivatives'<br>* das ganze Dokument *<br>--- | 1,5-8 | |
| X | JOURNAL OF MEDICINAL CHEMISTRY.<br>Bd. 17, Nr. 9, September 1974, WASHINGTON US<br>Seiten 1033 - 1035;<br>J. D. WEAVER ET AL.: 'Cycloserine Carbamates'<br>* das ganze Dokument *<br>---<br>-/-- | 1,5-8 | |

Der vorliegende Recherchenbericht wurde für alle Patentansprüche erstellt

| Recherchenort | Abschlußdatum der Recherche | Prüfer |
|---|---|---|
| DEN HAAG | 17 JANUAR 1992 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 91 11 7896
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | MONATSHEFTE FUR CHEMIE.<br>Bd. 89, Nr. 4-5, 24. Oktober 1958, WIEN AT<br>Seiten 627 - 628;<br>H.BRETSCHNEIDER: 'Synthese und antibakterielle Eigenschaften des D,L-N,N-Dimethylcycloserins'<br>* das ganze Dokument *<br>--- | 1,5-8 | |
| X | TETRAHEDRON, (INCL. TETRAHEDRON REPORTS)<br>Bd. 23, 1967, OXFORD GB<br>Seiten 65 - 76;<br>G.W.A.MILNE: 'The Nuclear Magnetic Resonance and Mass Spectra of Derivatives of Cycloserine'<br>* Seite 71 *<br>--- | 1 | |
| X | CHEMICAL ABSTRACTS, vol. 54, no. 9, 10. Mai 1960, Columbus, Ohio, US;<br>abstract no. 8852H,<br>JIRI SMRT: '4-Amino-3-isoxazolidinones'<br>Seite 8852 ;<br>* Zusammenfassung *<br>& CS-A-87 649 (JIRI SMRT) 15. September 1958<br>--- | 1,5-8 | RECHERCHIERTE SACHGEBIETE (Int. Cl.5) |
| X | CHEMICAL ABSTRACTS, vol. 56, no. 8, 16. April 1962, Columbus, Ohio, US;<br>abstract no. 9222G,<br>M .A. BREGER: 'Biological activity of cycloserine and some of its analogs and homologs'<br>Seite 9222 ;<br>* Zusammenfassung *<br>& ANTIBIOTIKI<br>Bd. 6, Nr. 9, 1961,<br>Seiten 26 - 29;<br>---<br>-/-- | 1,5-8 | |

Der vorliegende Recherchenbericht wurde für alle Patentansprüche erstellt

| Recherchenort | Abschlußdatum der Recherche | Prüfer |
|---|---|---|
| DEN HAAG | 17 JANUAR 1992 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 91 11 7896
Seite 3

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| X | CHEMICAL ABSTRACTS, vol. 68, no. 21, 20. Mai 1968, Columbus, Ohio, US; abstract no. 93173A, L. P.SASHCHENKO: 'Inhibition of L-glutamate decarboxylase by derivatives of hydroxylamine and related compounds' Seite 8977 ; * Zusammenfassung * & BIOKHIMIYA Bd. 33, Nr. 1, 1968, Seiten 142 - 147; ----- | 1,5-8 | |
| | | | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| DEN HAAG | 17 JANUAR 1992 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)